# FASCICULE DE BREVET EUROPEEN

(11) **EP 1 234 811 B1**
(45) Date de publication et mention de la délivrance du brevet: **27.10.2004**
(21) Numéro de dépôt: 02290396.7
(22) Date de dépôt: 19.02.2002
(51) Int. Cl.: C07C 41/16, C07C 43/20, C07C 45/71, C07C 49/84

(54) **Procédé de synthèse de mono-éthers d'aryle et d'alkyle**
Verfahren zur Herstellung von Monoarylalkylethern
Process for the synthesis of mono aryl alkyl ethers

(30) Priorité: 23.02.2001 FR 0102493
(43) Date de publication de la demande: 28.08.2002
(73) Titulaire: SNPE, 75181 Paris Cedex 04 (FR)
(72) Inventeur: Borredon, Elisabeth, 31170 Tournefeuille (FR); Gaset, Antoine, 31000 Toulouse (FR); Le Gars, Pierre, 31078 Toulouse (FR); Ouk, Samedy, 31400 Toulouse (FR); Thiebaud-Roux, Sophie, 31240 L'Union (FR)
(74) Mandataire: Ahner, Francis

(56) Documents cités:
- US-A- 4 192 949
- US-A- 4 254 276
- Y. LEE: "Convenient O-methylation of phenols with dimethyl carbonate" SYNLETT, octobre 1998 (1998-10), pages 1063-1064, XP002182473 STUTTGART DE
- A. BOMBEN: "A continuous-flow O-methylation of phenols with dimethyl carbonate in a continuously fed stirred tank reactor" IND. ENG. CHEM. RES., vol. 38, no. 5, 1999, pages 2075-2079, XP002182474
- DATABASE WPI Section Ch, Week 8748 Derwent Publications Ltd., London, GB; Class C03, AN 1987-339779 XP002182475 & JP 62 246533 A (DAICEL CHEM IND), 27 octobre 1987 (1987-10-27)

## Description

La présente invention concerne un procédé de synthèse d'éthers d'aryle et d'alkyle.

Plus particulièrement, elle concerne un procédé de synthèse améliorée des éthers d'aryle et d'alkyle par O-alkylation des composés phénoliques correspondants.

Les éthers d'aryle et d'alkyle sont des intermédiaires très utiles en particulier pour la préparation de colorants, d'agents phytosanitaires et de parfums. Leurs applications sont notamment rapportées dans Ullmann Enzyklopàdie der Technischen Chemie, volume 13, pages 450-453 et volume 14, pages 760-763. De très nombreux procédés de préparation ont par conséquent été proposés.

Certains consistent à alkyler les dérivés phénoliques avec des halogènures d'alkyle ou des sulfates d'alkyle comme indiqué dans le Pure & Applied Chemistry, volume 68, n° 2, pages 367-375 (1996). Mais ces procédés présentent de nombreux inconvénients. Certains réactifs comme le sulfate de diméthyle sont très toxiques. De plus, l'acide libéré au cours de la réaction doit être neutralisé, or certains phénols sont très sensibles aux agents neutralisants.

D'autres procédés utilisent comme agent alkylant un alcool, le méthanol par exemple. La réaction est effectuée à de très hautes températures, supérieures à 250°C. Dans la plupart des cas, cette réaction n'est pas sélective, des produits secondaires de C-alkylation sont formés. Lorsqu'elle est sélective, le taux de conversion est faible. Un tel procédé est décrit dans le volume 44 de Catalysis Today, pages 253-258 (1998).

Des procédés d'alkylation par des carbonates de dialkyle ont alors été envisagés, notamment des procédés de O-alkylation par du carbonate de diméthyle. Le catalyseur utilisé est choisi parmi les sels d'amines tertiaires, les diamines, les sels d'ammonium quaternaire, les phosphines tertiaires. De tels procédés sont par exemple décrits dans le brevet US 4 192 949. Mais, la température, la pression et la durée de réaction de ces synthèses réalisées en réacteur fermé restent élevées, ce qui est très pénalisant au niveau industriel.

Afin de préparer des éthers d'aryle et d'alkyle dans des conditions plus douces, les auteurs de l'article paru dans Synthesis, volume 5, pages 382-383 (1986), préconisent l'utilisation du carbonate de potassium avec un co-catalyseur, l'éther couronne 18-6. Il s'agit d'une catalyse par transfert de phase solide/liquide. La réaction est effectuée à pression atmosphérique et à 100°C mais la haute toxicité et le coût élevé du co-catalyseur de transfert de phase, l'éther couronne, sont des inconvénients majeurs. De plus, la vitesse moyenne de formation de l'éther par mole de catalyseur est de l'ordre de 0,03 mole par heure, ce qui est faible.

Il a alors été proposé, afin de travailler avec des réactifs non toxiques, un autre système catalytique. Il s'agit toujours d'une catalyse par transfert de phase. Un tel procédé est décrit dans Industrial & Engineering Chemistry Research, volume 27, pages 1565-1571 (1988). Tundo et al. utilisent comme système catalytique du polyéthylèneglycol adsorbé sur un lit fixe solide constitué soit de carbonate de potassium, soit de billes d'α-alumine. Dans ce dernier cas, du carbonate de potassium est également adsorbé sur les billes d'α-alumine. Ce procédé présente l'inconvénient d'utiliser un système catalytique complexe, avec au moins deux constituants, le polyéthylène glycol et le carbonate de potassium. La présence simultanée de ces deux constituants est indispensable pour obtenir un bon rendement. En effet, à titre comparatif, lorsque le système catalytique est constitué de billes d'α-alumine recouvertes uniquement de 5 % en poids de carbonate de potassium, la conversion du phénol en anisole, comme indiqué sur la figure 4, est de 25 %, ce qui est très faible. De plus, l'hétérogénéité du milieu réactionnel réduit l'efficacité de la réaction. La vitesse moyenne de formation de l'éther par mole de catalyseur n'est alors que de 0,13 mole par heure.

Un procédé continu semblable a été envisagé par Bomben et al. dans un article paru dans Industrial & Engineering Chemistry Research, volume 38, pages 2075-2079 (1999). Les auteurs de cet article utilisent, comme système catalytique, un lit catalytique agité constitué de polyéthylèneglycol et de carbonate de potassium. Les inconvénients liés à un tel procédé restent toujours la complexité du système catalytique à deux composants et la faible valeur de la vitesse moyenne de formation de l'éther par mole de catalyseur, qui n'est que de 0,7 mol/mol.h.

Un autre procédé, décrit dans la demande japonaise JP 06145091, consiste à faire réagir un composé phénolique tel que le phénol ou l'hydroquinone et un carbonate d'alkyle. Le catalyseur utilisé est un sel de métal alcalin, notamment le carbonate de potassium. La réaction a lieu obligatoirement en présence d'un solvant organique azoté, tel que la pyridine, un formamide ou un acétamide d'alkyle. Les auteurs ont démontré, dans l'exemple comparatif 1, que le rendement était nul lorsque la réaction était effectuée sans solvant. De plus, le temps de réaction reste long et les rendements sont peu élevés.

Dans l'article "Convenient O-Methylation of PhenolS with Dimethyl Carbonate, Synlett, october 1998, p.1063-1064", il est décrit un procédé qui consiste à faire réagir des phénols avec du diméthylcarbonate en présence de carbonates alcalins sans solvant mais le procédé utilisé est un procédé de laboratoire difficilement transposable dans l'industrie. Les réactifs sont placés dans un tube scellé. La réaction a donc lieu sous pression élevée. Les durées de réaction et les rendements ne sont convenables qu'avec le carbonate de césium. En présence de carbonate de potassium, le rendement n'est que de 69 % après 22 heures de réaction. En présence de carbonate de sodium, 7 jours sont nécessaires pour obtenir un rendement de 91 %.

L'homme du métier est donc toujours à la recherche d'un procédé de synthèse d'éthers d'aryle et d'alkyle peu coûteux, sélectif, avec un bon rendement et dont les conditions de réaction sont douces, notamment en ce qui concerne la pression et la température.

La présente invention a pour objet un tel procédé.

Elle concerne un procédé de synthèse de mono-éthers d'aryle et d'alkyle par réaction d'un composé phénolique contenant un ou plusieurs groupes hydroxyles fixés sur le système cyclique aromatique et d'un carbonate de dialkyle, caractérisé en ce que ledit procédé est effectué sans solvant, à une pression comprise entre 0,93.10⁵ Pa et 1,07.10⁵ Pa, à une température comprise entre 100°C et 200°C, en présence d'un catalyseur choisi dans le groupe constitué par les carbonates alcalins et les hydroxydes alcalins et en ce que le carbonate de dialkyle est ajouté progressivement dans le milieu réactionnel.

Ce procédé présente l'avantage d'être simple et peu coûteux, et permet d'obtenir des éthers avec un très bon rendement.

En effet, il s'agit d'un procédé sans solvant, n'utilisant qu'un seul catalyseur. L'utilisation d'un nombre limité de constituants réduit donc les coûts. De plus, les conditions opératoires, notamment en ce qui concerne la pression et la température, sont faciles à mettre en oeuvre industriellement.

La réaction est sélective. En effet, lorsque le composé phénolique ne contient qu'un groupe hydroxyle sur le système cyclique aromatique, seul l'éther d'aryle et d'alkyle correspondant est obtenu, et sans formation de produits secondaires.

Lorsque le composé phénolique contient deux ou plusieurs groupes hydroxyles fixés sur le système cyclique aromatique, un mono-éther d'aryle et d'alkyle est obtenu majoritairement. Seules de faibles quantités de poly-éthers sont obtenues.

Un autre avantage de ce procédé est la valeur élevée de la vitesse moyenne de formation de l'éther par rapport à la quantité de catalyseur utilisée, qui est de l'ordre de 2 à 6 moles d'éther formées par heure et par mole de catalyseur utilisée. Avec les procédés déjà existants, la vitesse moyenne était inférieure ou égale à 1 mole d'éther formée par heure et par mole de catalyseur.

Ce procédé permet d'obtenir de façon sélective une large gamme de mono-éthers d'aryle et d'alkyle à partir d'un carbonate de dialkyle et d'un composé phénolique.

De façon préférée, le composé phénolique est choisi parmi les composés de formule (I) 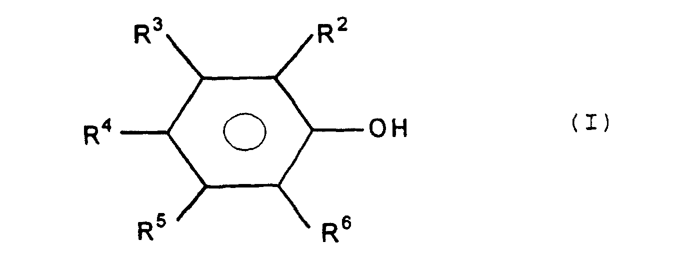 dans laquelle R², R³, R⁴, R⁵, R⁶ identiques ou différents représentent chacun
- un atome d'hydrogène,
- un radical alkyle en C₁ à C₂₀, saturé ou insaturé, substitué ou non,
- un groupe aryle ou aralkyle substitué ou non,
- un atome d'halogène,
- un groupe nitrile, nitro, un groupe de formule : 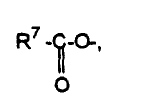 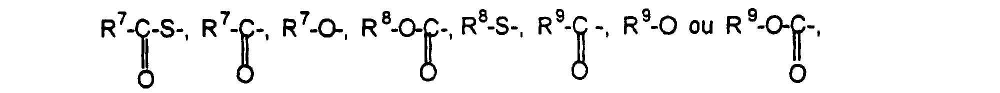
dans laquelle R⁷ est un radical aliphatique en C₁ à C₂₀, aralkyle en C₇ à C₁₂ ou aromatique en C₆ à C₁₄, R⁸ est un radical aliphatique en C₁ à C₂₀, aralkyle en C₇ à C₁₂ ou aromatique en C₆ à C₁₄, et R⁹ est un atome d'hydrogène,
- deux radicaux adjacents, par exemple R²R³ ou R³R⁴ ou R⁴R⁵ ou R⁵R⁶ pouvant être reliés entre eux pour former un cycle aliphatique saturé ou non, un cycle aromatique ou un hétérocycle saturé ou non, substitués ou non par les groupes tels que décrits pour R² à R⁶.
   Les substituants des radicaux R² à R⁶ sont en particulier choisis parmi les atomes d'halogtène, les groupes nitrile, nitro, les groupes de formule 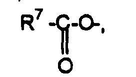 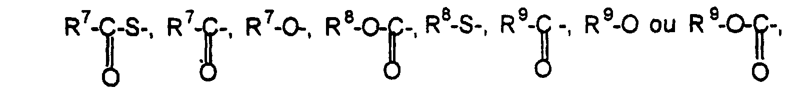
dans lesquelles R⁷, R⁸ et R⁹ ont la signification précédente.

Les composés de formule I sont des composés connus que l'on trouve dans le commerce ou que l'on prépare selon des méthodes connues.

Les composés phénoliques préférés sont le phénol, le p-crésol, le 4-chlorophénol, le 2-naphtol, la 4-hydroxybenzophénone, la 2,4-dihydroxybenzophénone et le catéchol.

Le carbonate de dialkyle est un carbonate dont les groupes alkyles, de préférence identiques, sont des groupes en C₁ à C₄. De préférence, le carbonate de dialkyle est choisi dans le groupe constitué par le carbonate de diméthyle et le carbonate de diéthyle, et mieux encore, le carbonate de dialkyle utilisé est le carbonate de diméthyle.

La quantité de carbonate de dialkyle utilisée est généralement comprise entre 0,9 et 5 moles, et de préférence de 1 à 2 moles par rapport au composé phénolique.

Le catalyseur utilisé est choisi dans le groupe constitué par les carbonates alcalins et les hydroxydes alcalins. Les carbonates alcalins comprennent à la fois les carbonates neutres alcalins et les hydrogénocarbonates alcalins.

De préférence, l'ion alcalin est le potassium et mieux encore le catalyseur utilisé est le carbonate neutre de potassium.

La quantité de catalyseur utilisée est comprise entre 0,01 et 0,1 mole par mole de composé phénolique et, de préférence, comprise entre 0,015 et 0,05 mole.

La réaction est effectuée à une pression comprise entre 0,93.10⁵ Pa et 1,07.10⁵ Pa, soit à une pression comprise entre 700 mm Hg et 800 mm Hg. Généralement, la pression atmosphérique locale est comprise dans cette gamme et la réaction est effectuée à cette pression. La température est comprise entre 100°C et 200°C, et, de préférence, comprise entre 140°C et 180°c.

Il s'agit d'un procédé sans solvant, c'est-à-dire sans l'apport d'un produit autre que les composés de la réaction.

On donne maintenant une mise en oeuvre de l'invention.

On place initialement dans le réacteur le composé phénolique, soit totalement soit en partie, le catalyseur et éventuellement du produit final, le composé phénolique déjà alkylé. Le milieu est hétérogène à température ambiante. On chauffe à une température comprise entre 100°C et 200°C, de préférence comprise entre 140°C et 180°C. Le milieu devient homogène à la température de réaction.

Le carbonate de dialkyle est ensuite introduit progressivement dans le milieu réactionnel. De façon préférée, une petite partie du carbonate de dialkyle est introduite dans le milieu puis on introduit le carbonate de dialkyle en continu avec un débit compris entre 1 et 20 mol/h par mole de catalyseur ou par ajouts discontinus très rapprochés dans le temps.

Lorsque seulement une partie de la quantité de composé phénolique est présente initialement dans le réacteur, l'autre partie est ajoutée progressivement dans le milieu réactionnel, de préférence simultanément à l'ajout de carbonate de dialkyle. Son débit est alors compris, de préférence, entre 0,5 mol/h et 20 mol/h par mole de catalyseur.

L'alcool produit lors de la réaction est distillé au fur et à mesure de sa formation. L'éther obtenu peut ensuite être purifié par distillation.

Ce procédé convient particulièrement bien pour la synthèse des mono-éthers d'aryle et de méthyle, obtenus par O-méthylation du composé phénolique correspondant, par l'action du carbonate de diméthyle sur le composé phénolique.

Des rendements très élevés, supérieurs à 95 % peuvent être obtenus par O-méthylation notamment à partir du p-crésol, du 4-chlorophénol et du 2-naphtol.

De bons rendements sont également obtenus à partir du phénol, de la 4-hydroxybenzophénone et de la 2,4-dihydroxybenzophénone.

Ce procédé convient aussi particulièrement bien pour la synthèse des mono-éthers d'aryle et d'éthyle, obtenus par O-éthylation du composé phénolique correspondant, par l'action du carbonate de diéthyle sur le composé phénolique. De bons rendements sont obtenus, notamment à partir du p-crésol.

Les exemples suivants illustrent, à titre non limitatif, des variantes de mise en oeuvre de l'invention.

### Exemple 1 : synthèse du 4-méthylanisole

La réaction est effectuée dans un réacteur de 500 ml. Ce réacteur est surmonté d'un système de réfrigération à 20°C. Le réacteur est équipé d'un thermomètre, d'un agitateur mécanique et d'un système d'alimentation tel que l'on puisse l'alimenter en continu tout au long de la réaction. On introduit dans le réacteur 130g de p-crésol, soit 1200 mmoles et 5,5g de carbonate de potassium, soit 40 mmoles, soit 3,3 % molaire par rapport au p-crésol.

On chauffe le milieu à 160°C et on maintient cette température tout au long de la synthèse.

On introduit alors 16,2g de carbonate de diméthyle, soit 180 mmol. On alimente ensuite le réacteur en continu avec du carbonate de diméthyle jusqu'à la consommation totale du p-crésol. Ainsi, pendant 7,5 heures, on alimente le réacteur avec un débit continu de 210 mmol/h de carbonate de diméthyle.

Le méthanol produit est distillé au fur et à mesure de sa formation. On obtient ensuite, après purification par distillation, 142,2g de 4-méthylanisole soit 1164 mmol, ce qui correspond à un rendement de 97 %.

La vitesse moyenne de formation du 4-méthylanisole pour la quantité de catalyseur utilisée est de 3,9 mol/mol.h.

### Exemple 2 : Synthèse du 4-méthylanisole avec introduction de p-crésol en continu.

Le montage utilisé est le même que celui de l'exemple 1. On introduit dans le réacteur 21 g de p-crésol, soit 194 mmol et 1,38g de carbonate de potassium, soit 10 mmol, soit 5,2 % molaire par rapport au p-crésol et 50g de méthylanisole, soit 409 mmol. On chauffe le milieu à 160°C. On maintient cette température tout au long de la synthèse.

On introduit 9,9g de carbonate de diméthyle, soit 110 mmol. Puis on alimente le réacteur avec des débits continus de 72 mmol/h de carbonate de diméthyle et de 60 mmol/h de p-crésol. Après 32 heures de réaction, on continue d'introduire seulement du carbonate de diméthyle avec le même débit, pendant 10 heures. Le méthanol est distillé au fur et à mesure de sa formation.

On obtient 303g de 4-méthylanisole, soit 2 480 mmol, ce qui correspond à un rendement de 98 %.

La vitesse moyenne de formation du 4-méthylanisole pour la quantité de catalyseur utilisée est de 4,9 mol/mol.h.

### Exemple 3 : synthèse du 4-chloroanisole

Le montage utilisé est le même que celui de l'exemple 1.

On introduit dans le réacteur 64,3 g de 4-chlorophénol, soit 500 mmoles et 2,8g de carbonate de potassium, soit 20 mmol, soit 4 % molaire par rapport au 4-chlorophénol.

On chauffe le milieu à 160°C et on maintient cette température tout au long de la synthèse.

On introduit alors 19,7g de carbonate de diméthyle, soit 219 mmol. On alimente ensuite le réacteur en continu avec du carbonate de diméthyle jusqu'à la consommation totale du 4-chlorophénol. Ainsi, pendant 4,5 heures on alimente le réacteur avec un débit continu de 150 mmol/h de carbonate de diméthyle.

Le méthanol dans le milieu réactionnel est distillé au fur et à mesure de sa formation.

On obtient 70,6g de 4-chloroanisole, soit 495 mmol, ce qui correspond à un rendement de 99 %.

La vitesse moyenne de formation du 4-chloroanisole pour la quantité de catalyseur utilisée est de 5,5 mol/mol.h.

### Exemple 4 : synthèse du 2-méthoxynaphtalène

Le montage utilisé est le même que celui de l'exemple 1.

On introduit dans le réacteur 72,1g de 2-naphtol, soit 500 mmol et 5,5g de carbonate de potassium, soit 40 mmol, soit 8 % molaire par rapport au 2-naphtol. On chauffe le milieu à 160°C et on maintient cette température tout au long de la synthèse.

On introduit alors 18,7g de carbonate de diméthyle, soit 207 mmol. On alimente ensuite le réacteur en continu avec du carbonate de diméthyle jusqu'à la consommation totale du 2-naphtol. Ainsi, pendant 6 heures on alimente le réacteur avec un débit continu de 150 mmol/h de carbonate de diméthyle.

Le méthanol produit est distillé au fur et à mesure de sa formation.

On obtient 75,9g de 2-methoxynaphtalène, soit 480 mmol, ce qui correspond à un rendement de 96 %.

La vitesse moyenne de formation du 2-méthoxynaphtalène pour la quantité de catalyseur utilisée est de 2 mol/mol.h.

### Exemple 5 : synthèse du 4-éthoxytoluène

Le montage utilisé est le même que celui de l'exemple 1.

On introduit dans le réacteur 54g de p-crésol, soit 500 mmol et 2,8g de carbonate de potassium, soit 20 mmol, soit 4 % molaire par rapport au p-crésol. On chauffe le milieu à 160°C et on maintient cette température tout au long de la synthèse.

On introduit alors 25,1g de carbonate de diéthyle, soit 212 mmoles. On alimente ensuite le réacteur en carbonate de diéthyle avec un débit continu de 50 mmol/h, pendant 12 heures.

L'éthanol produit est distillé au fur et à mesure de sa formation.

On obtient 63,8 g de 4-éthoxytoluène, soit 469 mmol, ce qui correspond à un rendement de 94 %.

La vitesse moyenne de formation du 4-etoxytoluène pour la quantité de catalyseur utilisée est de 2 mol/mol.h.

### Exemple 6 : synthèse de l'anisole

Le montage utilisé est le même que celui de l'exemple 1.

On introduit dans le réacteur 47g de phénol, soit 500 mmol et 2,8g de carbonate de potassium, soit 20 mmol, soit 4 % molaire par rapport au phénol. On chauffe le milieu à 150-160°C et on maintient cette température tout au long de la synthèse.

On introduit alors 9,9g de carbonate de diméthyle, soit 110 mmol. On alimente ensuite le réacteur en carbonate de diméthyle avec un débit continu de 80 mmoles/h pendant 8 heures.

Le méthanol produit est distillé au fur et à mesure de sa formation. On obtient 41,6g d'anisole, soit 385 mmol, ce qui correspond à un rendement de 77 %.

La vitesse moyenne de formation de l'anisole pour la quantité de catalyseur utilisée est de 2,4 mol/mol.h.

### Exemple 7 : synthèse de la 2-hydroxy-4-méthoxybenzophénone

Le montage utilisé est le même que celui de l'exemple 1.

On introduit dans le réacteur 107,1g de 2,4-dihydroxybenzophénone, soit 500 mmol et 2,8g de carbonate de potassium, soit 20 mmol, soit 4 % molaire par rapport à la 2,4-dihydroxybenzophénone. On chauffe le milieu à 160°C et on maintient cette température tout au long de la synthèse.

On introduit alors 22,4g de carbonate de diméthyle, soit 249 mmol. On alimente ensuite le réacteur en carbonate de diméthyle avec un débit continu de 60 mmol/h pendant 10 heures.

Le méthanol produit est distillé au fur et à mesure de sa formation.

On obtient 412 mmol de 2-hydroxy-4-méthoxybenzophénone, ce qui correspond à un rendement de 82 %. Seules des traces du diéther sont obtenues.

La vitesse moyenne de formation de la 2-hydroxy-4-méthoxybenzophénone pour la quantité de catalyseur utilisée est de 2 mol/mol.h.

### Exemple 8 :Synthèse du guaïcol

Le montage utilisé est le même que celui de l'exemple 1.

On introduit dans le réacteur 55g de catéchol, soit 500 mmol et 5,5g de carbonate de potassium, soit 40 mmol, soit 8 % molaire par rapport au catéchol.

On chauffe le milieu à 160°C et on maintient cette température tout au long de la synthèse.

On introduit alors 18g de carbonate de diméthyle, soit 200 mmol. On alimente ensuite le réacteur en carbonate de diméthyle avec un débit continu de 100 mmol/h pendant 3 heures.

Le méthanol produit est distillé au fur et à mesure de sa formation.

On obtient 29,8g de guaïcol, soit 240 mmol, le composé monométhyléther, ce qui correspond à un rendement de 48 % et on obtient seulement 6 % de diméthyléther, le tératrol.

## Revendications

1. Procédé de synthèse de mono-éthers d'aryle et d'alkyle par réaction d'un composé phénolique contenant un ou plusieurs groupes hydroxyles fixés sur le système cyclique aromatique et d'un carbonate de dialkyle, en présence d'un catalyseur choisi dans le groupe constitué par les carbonates alcalins et les hydroxydes alcalins, sans solvant ledit procédé étant effectué à une pression comprise entre 0,93.10⁵ Pa et 1,07.10⁵ Pa, à une température comprise entre 100°C et 200°C et le carbonate de dialkyle étant ajouté progressivement dans le milieu réactionnel.

2. Procédé selon la revendication 1, **caractérisé en ce que** ledit composé phénolique est un composé de formule (I) 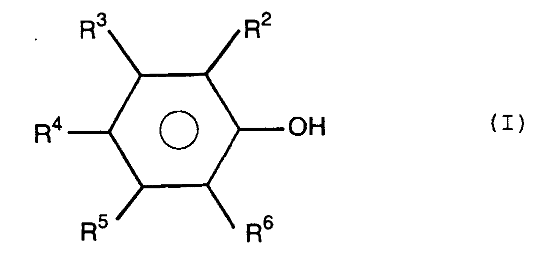 dans laquelle R², R³, R⁴, R⁵, R⁶ identiques ou différents représentent chacun
- un atome d'hydrogène,
- un radical alkyle en C₁ à C₂₀, saturé ou insaturé, substitué ou non,
- un groupe aryle ou aralkyle substitué ou non,
- un atome d'halogène,
- un groupe nitrile, nitro, un groupe de formule : 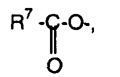 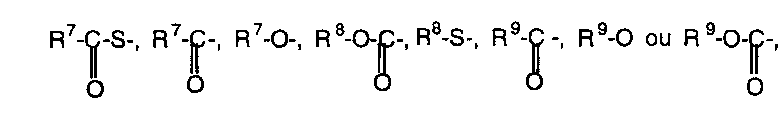
dans laquelle R⁷ est un radical aliphatique en C₁ à C₂₀, aralkyle en C₇ à C₁₂ ou aromatique en C₆ à C₁₄, R⁸ est un radical aliphatique en C₁ à C₂₀, aralkyle en C₇ à C₁₂ ou aromatique en C₆ à C₁₄ et R⁹ est un atome d'hydrogène,
- deux radicaux adjacents, R²R³ ou R³R⁴ ou R⁴R⁵ ou R⁵R⁶ pouvant être reliés entre eux pour former un cycle aliphatique saturé ou non, un cycle aromatique ou un hétérocycle saturé ou non, substitués ou non par les groupes tels que décrits pour R² à R⁶.

3. Procédé selon l'une quelconque des revendications 1 ou 2, **caractérisé en ce que** le carbonate de dialkyle est choisi dans le groupe constitué par le carbonate de diméthyle et le carbonate de diéthyle.

4. Procédé selon l'une quelconque des revendications 1 à 3, **caractérisé en ce que** le catalyseur est le carbonate neutre de potassium.

5. Procédé selon l'une quelconque des revendications 1 à 4, **caractérisé en ce que** la quantité de catalyseur utilisée est comprise entre 0,01 et 0,1 mole par mole de composé phénolique.

6. Procédé selon l'une quelconque des revendications 1 à 5, **caractérisé en ce que** la quantité de carbonate de dialkyle est comprise entre 0,9 et 5 moles par mole de composé phénolique.

7. Procédé selon l'une quelconque des revendications 1 à 6, **caractérisé en ce que** la température de réaction est comprise entre 140°C et 180°C.

8. Procédé selon l'une quelconque des revendications 1 à 7, **caractérisé en ce que** le carbonate de dialkyle est ajouté en continu dans le milieu réactionnel avec un débit compris entre 1 et 20 mol/h par mole de catalyseur.

9. Procédé selon l'une quelconque des revendications 1 à 8, **caractérisé en ce qu'**une partie de la quantité de composé phénolique est présente initialement dans le réacteur, et que l'autre partie est ajoutée progressivement dans le milieu réactionnel.

## Patentansprüche

1. Verfahren zur Synthese von Aryl- und Alkylmonoethern durch Umsetzung einer phenolischen Verbindung, die eine oder mehrere Hydroxylgruppen enthält, die an dem cyclischen aromatischen System angebracht sind, und eines Dialkylcarbonats in Anwesenheit eines Katalysators, der aus der Gruppe ausgewählt ist, die aus Alkalicarbonaten und Alkalihydroxiden besteht, ohne Lösungsmittel, wobei das Verfahren bei einem Druck zwischen 0,93·10⁵ Pa und 1,07·10⁵ Pa einschließlich bei einer Temperatur zwischen 100 °C und 200 °C einschließlich durchgeführt wird und das Dialkylcarbonat nach und nach in das Reaktionsmedium gegeben wird.

2. Verfahren nach Anspruch 1, **dadurch gekennzeichnet, dass** die phenolische Verbindung eine Verbindung der Formel (I) ist 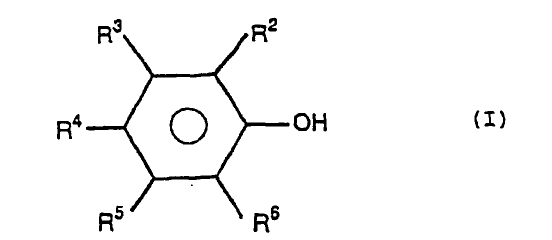 in der R², R³, R⁴, R⁵, R⁶, identisch oder verschieden, jeweils
- ein Wasserstoffatom,
- einen gesättigten oder ungesättigten, substituierten oder unsubstituierten C₁bis C₂₀-Alkylrest,
- eine substituierte oder unsubstituierte Aryl- oder Aralkylgruppe,
- ein Halogenatom,
- eine Nitril-, Nitrogruppe, eine Gruppe der Formel: 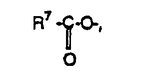 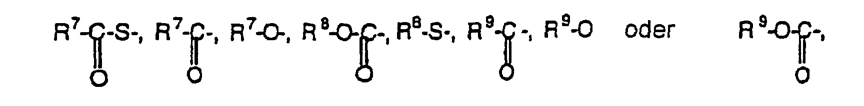 in der R⁷ ein aliphatischer C₁- bis C₂₀-, ein C₇- bis C₁₂-Aralkyl- oder ein aromatischer C₆- bis C₁₄-Rest ist, R⁸ ein aliphatischer C₁- bis C₂₀-, ein C₇- bis C₁₂-Aralkyl- oder ein aromatischer C₆- bis C₁₄-Rest ist und R⁹ ein Wasserstoffatom ist,
darstellen,
- zwei benachbarte Reste, R²R³ oder R³R⁴ oder R⁴R⁵ oder R⁵R⁶, miteinander verbunden sein können, um einen gesättigten oder ungesättigten aliphatischen Cyclus, einen aromatischen Cyclus oder einen gesättigten oder ungesättigten Heterocyclus, die durch die Gruppen, wie für R² bis R⁶ beschrieben, substituiert sein können oder nicht, zu bilden.

3. Verfahren nach irgendeinem der Ansprüche 1 oder 2, **dadurch gekennzeichnet, dass** das Dialkylcarbonat aus der Gruppe ausgewählt ist, die aus Dimethylcarbonat und Diethylcarbonat besteht.

4. Verfahren nach irgendeinem der Ansprüche 1 bis 3, **dadurch gekennzeichnet, dass** der Katalysator neutrales Kaliumcarbonat ist.

5. Verfahren nach irgendeinem der Ansprüche 1 bis 4, **dadurch gekennzeichnet, dass** die verwendete Katalysatormenge zwischen 0,01 und 0,1 Mol einschließlich pro Mol phenolische Verbindung liegt.

6. Verfahren nach irgendeinem der Ansprüche 1 bis 5, **dadurch gekennzeichnet, dass** die Menge an Dialkylcarbonat zwischen 0,9 und 5 Mol einschließlich pro Mol phenolische Verbindung liegt.

7. Verfahren nach irgendeinem der Ansprüche 1 bis 6, **dadurch gekennzeichnet, dass** die Reaktionstemperatur zwischen 140 °C und 180 °C einschließlich liegt.

8. Verfahren nach irgendeinem der Ansprüche 1 bis 7, **dadurch gekennzeichnet, dass** das Dialkylcarbonat kontinuierlich mit einer Zugabegeschwindigkeit zwischen 1 und 20 Mol/h einschließlich pro Mol Katalysator in das Reaktionsmedium gegeben wird.

9. Verfahren nach irgendeinem der Ansprüche 1 bis 8, **dadurch gekennzeichnet, dass** ein Teil der Menge der phenolischen Verbindung anfänglich in dem Reaktor vorliegt und dass der andere Teil nach und nach in das Reaktionsmedium gegeben wird.

## Claims

1. Synthesis process for aryl and alkyl mono-ethers by reaction of a phenolic compound containing one or several hydroxyl groups fixed on the cyclic aromatic system and a dialkyl carbonate, in the presence of a catalyst chosen from the group composed of alkaline carbonates and alkaline hydroxides, without solvent, the said process being carried out at a pressure of between 0.93 x 10⁸ and 1.07 x 10⁵ Pa, at a temperature of between 100°C and 200°C, and the dialkyl carbonate being progressively added into the reaction medium.

2. Process according to claim 1, **characterised in that** the said phenolic compound is a compound with formula (I) 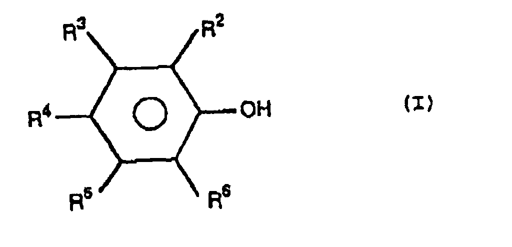 in which R², R³, R⁴, R⁵, R⁶ are identical or different and each represents:
- a hydrogen atom,
- an alkyl radical in C₁ to C₂₀, saturated or unsaturated, substituted or not,
- an aryl or aralkyl group, substituted or not,
- a halogen atom,
- a nitrile, nitro group, or a group with formula: 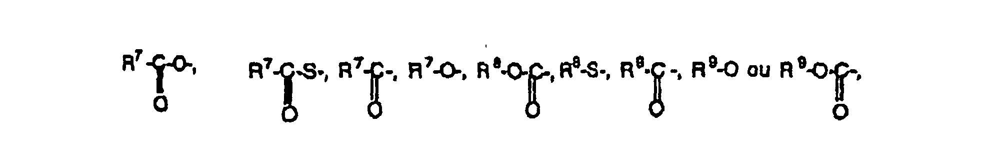
in which R⁷ is an aliphatic radical in C₁ to C₂₀, an aralkyl radical in C₇ to C₁₂, or an aromatic radical in C₆ to C₁₄, R⁸ is an aliphatic radical in C₁ to C₂₀, an aralkyl radical in C₇ to C₁₂ or an aromatic radical in C₆ to C₁₄ and R⁹ is a hydrogen atom,
- two adjacent radicals R²R³ or R³R⁴ or R⁴R⁵ or R⁵R⁶ that can be connected together to form a saturated or unsaturated aliphatic cycle, an aromatic cycle or a saturated or unsaturated heterocycle, substituted or not substituted by groups as described for R² to R⁶.

3. Process according to either of claims 1 or 2, **characterised in that** the dialkyl carbonate is chosen from the group composed of dimethyl carbonate and diethyl carbonate.

4. Process according to any one of claims 1 to 3, **characterised in that** the catalyst is neutral potassium carbonate.

5. Process according to any one of claims 1 to 4, **characterised in that** the quantity of catalyst used is between 0.01 and 0.1 mole per mole of phenolic compound.

6. Process according to any one of claims 1 to 5, **characterised in that** the quantity of dialkyl carbonate is between 0.9 and 5 moles per mole of phenolic compound.

7. Process according to any one of claims 1 to 6, **characterised in that** the reaction temperature is between 140°C and 180°C.

8. Process according to any one of claims 1 to 7, **characterised in that** the dialkyl carbonate is added continuously into the reaction medium at a flow rate of between 1 and 20 mol/h per mole of catalyst.

9. Process according to any one of claims 1 to 8, **characterised in that** part of the quantity of phenolic compound is initially present in the reaction vessel, and the other part is added into the reaction medium progressively.
